# EUROPEAN PATENT SPECIFICATION

(11) **EP 2 255 191 B1**
(45) Date of publication and mention of the grant of the patent: **06.01.2016**
(21) Application number: 09713099.1
(22) Date of filing: 20.02.2009
(51) Int. Cl.: G01N 33/543, G01N 33/564

(54) **ASSAY METHOD FOR ANTIBODIES AGAINST CYCLIC CITRULLINATED PEPTIDE**
TESTVERFAHREN FÜR ANTIKÖRPER GEGEN CYCLISCHES CITRULLINIERTES PEPTID
PROCÉDÉ D'ANALYSE POUR DES ANTICORPS DIRIGÉS CONTRE UN PEPTIDE CITRULLINÉ CYCLIQUE

(30) Priority: 20.02.2008 GB 0803107; 18.04.2008 US 46082 P
(43) Date of publication of application: 01.12.2010
(73) Proprietor: Axis-Shield Diagnostics Ltd., Dundee DD2 1XA, Scotland (GB)
(72) Inventor: MILNE, Ken, Dundee DD2 1XA (GB); PRITCHARD, David, Dundee DD2 1XA (GB); SUNDREHAGEN, Erling, 0504 Oslo (NO)
(74) Representative: Goddard, Christopher Robert
(86) International application number: PCT/GB2009/000470
(87) International publication number: WO 2009/103988

(56) References cited:
- EP-A- 1 882 946
- WO-A-01/46222
- BAS S ET AL: "Diagnostic tests for rheumatoid arthritis: comparison of anti-cyclic citrullinated peptide antibodies, anti-keratin antibodies and IgM rheumatoid factors" RHEUMATOLOGY, OXFORD UNIVERSITY PRESS, LONDON, GB, vol. 41, no. 7, 1 July 2002 (2002-07-01), pages 809-814, XP002333658 ISSN: 1462-0324
- SHOVMAN O ET AL: "The diagnostic utility of anti-cyclic citrullinated peptide antibodies, matrix metalloproteinase-3, rheumatoid factor, erythrocyte sedimentation rate, and C-reactive protein in patients with erosive and non-erosive rheumatoid arthritis." CLINICAL & DEVELOPMENTAL IMMUNOLOGY SEP 2005, vol. 12, no. 3, September 2005 (2005-09), pages 197-202, XP002523636 ISSN: 1740-2522
- LEE D M ET AL: "Clinical utility of the anti-CCP assay in patients with rheumatic diseases.", ANNALS OF THE RHEUMATIC DISEASES, vol. 62, no. 9, September 2003 (2003-09), pages 870-874, ISSN: 0003-4967

## Description

The present invention relates to *in vitro* assay methods for detecting existence of, potential for, or propensity to rheumatoid arthritis (RA) in a subject. The invention relates especially to automated assays and in particular, to assay methods for assessing rheumatoid arthritis (RA) comprising measuring the existence or level of endogenous antibodies with specificity for cyclic citrullinated peptide (anti-CCP) in a subject. The invention also relates to methods for measuring the existence or level of endogenous antibodies with specificity for cyclic citrullinated peptide (anti-CCP) in a subject

Rheumatoid Arthritis (RA) is a common, systemic autoimmune disease affecting between 0.5-1% of the adult population. RA is characterised by inflammation of the synovial joints. This can lead to progressive joint destruction and consequently impair the quality of life. It is generally accepted that early intervention is vital in preventing irreversible joint damage and therefore it is important to diagnose RA as early in the disease course as possible. The 1987 American College of Rheumatology (ACR) classification is widely used in the diagnosis of RA, despite the fact they are not well suited for the diagnosis of early Rheumatoid Arthritis (RA). This is due to the fact that the ACR criteria rely heavily on the expression of clinical symptoms and these are often not manifest in early RA. Ideally a highly specific and sensitive serological marker for RA is required so that rheumatologists can identify those patients who would benefit as candidates for aggressive therapeutic regimes. Presently, the Rheumatoid Factor (RF) test is commonly used as a serological marker for RA, although it is accepted that the test lacks specificity and is also often absent in the disease (seronegative RA).

Over the last few years a novel antibody marker has been described which is reported to have high specificity (>95%) and sensitivity (80%) for RA patients. A subject having RA has been found to develop endogenous auto-antibodies to cyclic citrullinated peptides (anti-CCP), and these are used as a marker in the diagnosis of early RA. Since the first report in 1998 that antibodies present in blood and reactive with synthetic peptides containing the amino acid citrulline are highly specific for RA, the measurement of anti-CCP antibodies in patient blood or serum or plasma has become the method of choice in the early and accurate diagnosis of this disease.

The assay for endogenous antibodies specific to CCP (anti-CCP) is as sensitive as the rheumatoid factor test but with much higher specificity. Additionally a positive test for anti-CCP can predict future development of RA in both asymptomatic individuals and in patients with undifferentiated arthritis. It has also been shown that antibody levels at presentation can correlate with progression to erosive disease. Earlier diagnosis and treatment, preferably within the first several months after onset of symptoms, may help prevent irreversible joint damage.

In the prior art a heterogeneous immunoassay has been proposed to measure anti-CCP antibodies. Such heterogeneous measurement is based on directly or indirectly coating CCP to a solid phase, incubating the solid phase with a sample known or suspected to comprise anti-CCP antibodies under conditions allowing for binding of anti-CCP antibodies to CCP, and directly or indirectly detecting the anti-CCP antibodies bound. A further assay format is the so-called double antigen bridge assay, wherein, in case of an anti-CCP measurement, CCPs are used both at the solid phase side as well as at the detection side of this immunoassay and the autoantibodies in a patient sample form a bridge between these "double" antigens. Typically, several washing steps are included while performing a heterogeneous immunoassay. Although such methods are effective, they are relatively slow and demanding in terms of reagents and equipment required. It is also difficult to automate such assays, and when automated the reliability is relatively low.

EP 1882946 relates to anti-CCP and interleukin 6 in the diagnosis of RA and WO 01/46222 discloses immunoassays for anti-CCP.

In view of the above, there is a clear need for methods, especially based on biochemical parameters, aiding in the assessment of rheumatoid arthritis. Furthermore, with the emergence of anti-CCP as the test of choice for the diagnosis or indication of potential or propensity to RA, there is a clear need for increasing numbers of patients to be tested for the presence of anti-CCP antibodies. This then provides a need for simpler, faster, higher sensitivity, more accurate, higher throughput and/or more automated assay methods for the detection and/or quantification of anti-CCP antibodies in a body sample.

The present inventors have now surprisingly established that by providing a homogeneous assay for anti-CCP antibodies in a body sample as indicated herein, the balance of some or all of the above factors may be improved and thus a more effective clinical assay provided.

In a first aspect, the present invention thus provides a method for assaying anti-CCP antibodies in a clinical sample, selected from blood, blood derivatives, serum, plasma, urine, cerebrospinal fluid, oral fluid, synovial fluid and emphysema fluid, said method comprising providing a homogenous assay format, contacting said sample with at least one reagent comprising at least one specific binder for anti-CCP antibodies, immobilised to monodisperse metal or polymer nanoparticles having a diameter of between 10 and 250 nm, whereby to form a solution or suspension of an anti-CCP-binding partner complex in a homogeneous sample mixture and detecting the presence or level of said anti-CCP-binding partner complex in a homogenous liquid phase, wherein said detection is carried out by turbidimetry, wherein in said method the sample and at least one reagent are mixed, a signal generated and that signal detected without any separation or washing steps involving phase separation and wherein the binder for anti-CCP is at least one CCP peptide antigen where two or more such peptides are bound to each particle.

The primary application of the assessment of anti-CCP antibodies of the present invention is currently to analyse the existence of, risk of, potential for, and/or propensity to RA in the subject from whom the clinical sample was taken.

In a second aspect, the present invention therefore provides a method for the assessment of the existence of; risk of; potential for; or propensity to RA in a subject, said method comprising assaying for anti-CCP in a body sample from said subject in a homogeneous assay according to the present invention, whereby to determine the level (e.g. the existence, non-existence or relative or absolute concentration) of anti-CCP antibodies in said sample, and correlating the thus-determined level with the existence of; risk of; potential for; or propensity to RA in said subject, wherein the existence of anti-CCP, or a concentration of anti-CCP above one or more threshold values is correlated with existence of, increased severity of, increased risk of, increased potential for, and/or increased propensity to RA, and non-existence of anti-CCP or concentration of anti-CCP below one or more threshold values is correlated with non-existence of, decreased severity of, decreased risk of, decreased potential for, and/or decreased propensity to RA.

In all aspects of the invention relating to assessing RA in a subject, the method may also optionally include the assessment of other biochemical markers, such as Rheumatoid Factor (RF) in combination with the assessment of anti-CCP, as indicated above. Each of the factors measured may then be used with appropriate weightings to determine the probability or degree of existence of, risk of, potential for, and/or propensity to RA. The use of two or more independent factors also allows as assessment of the confidence which may be placed in the prediction. If all factors agree, for example then a high confidence exists. If, however, the factors disagree, then the tendency of each individual factor to provide false positives or false negatives may be taken into account (for example, it is known that around 20% of RA sufferers are negative for RF).

In the method of the second aspect of the invention in a patient receiving or having received treatment for RA, non-existence of anti-CCP, or concentration below at least one threshold value (e.g. an absolute or relative value, such as those indicated herein, or a value established from a previous assay by the present method on the same subject, including any correction factor for the expected progress of the disease with or without treatment) may be taken to indicate effective treatment. Conversely, existence of anti-CCP, or concentration above at least one threshold value (e.g. an absolute or relative value, such as those indicated herein, or a value established from a previous assay by the present method on the same subject, including any correction factor for the expected progress of the disease with or without treatment) may be taken to indicate less effective or ineffective treatment. The methods will thus also aid in monitoring the efficacy of treatment in patients suffering from RA or prophylaxis in subjects with a high risk of or propensity for suffering from RA.

In a further aspect, the present invention also provides a kit for performing the methods according to the present invention, comprising at least one specific binder for anti-CCP antibody immobilised to monodisperse metal or polymer nanoparticles having a diameter of between 10 and 250 nm wherein the binder for anti-CCP is at least one CCP peptide antigen and wherein two or more such peptides are bound to each particle. Kits of the invention may optionally include auxiliary reagents for performing the measurement.

Citrullinated peptides are antigens for important autoantibodies as found in the sera of patients with RA. The autoantibodies with the greatest clinical potential for RA are the anti-citrullinated protein antibodies directed to citrulline containing epitopes. Citrulline is a non-standard amino acid generated by post-translational modification of arginine residues by the enzyme peptidylarginine deiminase (PAD) - this process is referred to as Citrullination.

The assay methods of the invention utilise at least one specific binder to generate a complex comprising the binder and anti-CCP antibodies from the sample. The complex is then detected. In a preferred embodiment anti-CCP is measured using one or more CCP as antigenic binder. Binding of anti-CCP antibodies comprised in a sample to the CCP antigen is then detected by appropriate means. Alternatively, or in combination with an antigenic binder, antibodies raised against the anti-CCP antibodies themselves (anti-anti-CCP) can also be used as specific binders.

As used herein, the terms "binding partner" and "specific binder" are used to indicate a binder, partner or ligand which binds specifically to a component of interest (generally anti-CCP). Such binders will generally show little or no binding affinity for other components of the sample, such as other peptides, proteins or antibodies. For example, the affinity for non anti-CCP antibodies from the same subject should be no more than 1/100^{th}, preferably no more than 1/1,000^{th}, and most preferably no more than 1/10,000^{th} of the affinity of the binder or ligand for anti-CCP. A relative affinity for non anti-CCP antibodies of 1/10⁴ to 1/10⁸ is particularly preferred.

The assays of the present invention are homogeneous, which provides considerable benefits in simplifying the method, easing automation, reducing the number of reagents and reaction steps, and improving reliability. This does, however, impose considerable demands on the assay design, because there is no scope for washing away excess or unbound reagents.

The present inventors have now appreciated that by appropriate binding and detection techniques, homogeneous assays for anti-CCP antibodies may be designed, allowing high sensitivity and reliability without requiring washing and separation of a heterogeneous phase.

The term "homogeneous" as used herein indicates that a sample reagent or other mixture is in a stable single fluid phase. Molecular solutions will thus be considered homogeneous, as will suspensions of particulates sufficiently small that they do not settle or float significantly out of the bulk fluid phase over a period of at least the time required to perform the experiment, preferably 1 hour, more preferably 3 hours. Most preferred are suspensions which are stable over longer periods, such as at least one week, preferably at least 1 month. For detection, such as by turbidimetry, the methods of the invention use homogeneous techniques. In this case, it is necessary only that the mixtures remain homogeneous for sufficient time to take an accurate measurement (typically from 20 seconds to 3 hours, more preferably between 2 and 60 minutes).

In correspondence to the above, the term "homogeneous assay" as used herein is an assay method in which the sample and at least one reagent are mixed, a signal generated and that signal detected without any separation or washing steps involving phase separation. Thus, at no point in the homogeneous assay methods of the present invention is the anti-CCP antibody component of the sample, or any complex comprising the anti-CCP antibody component separated from the bulk liquid phase by binding or capture onto a solid support. There is thus no need for any phase-separation step and the process of the method is significantly simplified. Pre-concentration of the anti-CCP by phase-separation is possible prior to the method of the invention, but is a less preferred embodiment, since this increases the number of steps and the overall complexity of the assay. Evidently, because there are no washing steps possible in such a homogeneous assay method, the method, the binders and the signal generating moieties must be selected with this in mind, as is described in detail herein.

Compared to automation of non-homogeneous techniques involving, for example a solid surface, automation of suitable homogeneous assays is relatively facile. Also, the resulting automated homogenous process is often more reliable being less prone, for example, to break down as simpler instrumentation is required. An automated homogeneous assay is also fast, allowing for a high throughput of samples, and is relatively cheap to run.

Various types of known assay format may be applied to give a homogeneous assay, and of particular value are those in which a signal is generated by bringing components of the sample together. The multi-valent (especially di-valent) nature of antibodies allows them to serve as cross-linking agents, thus bringing their binders together in pairs, or where at least some of the specific binders are also multi-valent, into larger aggregates. It is preferred, therefore, that the anti-CCP-binding partner complex is detectable by a means which does not significantly detect the antibodies or any of the binding partners alone. Suitable methods include fluorescence resonance energy transfer (FRET - where the two fluorophores are separately attached to two anti-CCP binding partners); scintillation proximity assays (SP - where the radiolabel and the scintillant are separately attached to two anti-CCP binding partners); fluorescence polarisation (FP - where tumbling and thus de-polarisation of a fluorophore attached to at least one specific binder is slowed by the formation of larger complexes); and most particularly aggregation, where the absorption or scattering of incident radiation (especially light, such as laser light) is increased by the aggregation of small particles and/or components in the sample to generate larger particles having a greater scattering effect than the sum of the effects of the small particles/components from which they are formed. Particle aggregation is typically measured by turbidimetry or nephelometry.

A highly preferred embodiment of the present invention is thus the assessment of anti-CCP antibodies by means of homogeneous assays formats involving the agglutination of particles (such as latex particles) coated with or attached to anti-CCP binders such as CCP peptides and/or anti-CCP binding aptamers. CCP peptides are most preferred.

The threshold values and reference range may be determined by a number of practical techniques. The basis of any threshold value must ultimately be testing of patients with a disease at various stages and comparison of those results with control groups of healthy volunteers. This is a well-known practice, and should preferably be conducted directly for the assay methods of the present invention in order to build up a profile of the relevant population groups in the areas where the tests are conducted. A preferred threshold value will be such that at least 80% of patients with clinically confirmed RA will provide samples generating a signal above the chosen value (preferably this will be at least 90%, more preferably at least 95%, such as 95% to substantially 100%). Similarly, a preferred threshold value will be chosen such that at least 80%, preferably, at least 90% and more preferably at least 95% (such as 95% to substantially 100%) of healthy control subjects will provide samples generating a signal above the threshold.

Since existing methods for the determination of anti-CCP and correlation of this with RA are available and are effective, and alternative or complementary approach to the above method is to calibrate the signal generated from the present method against the scale of one of more existing anti-CCP RA assay methods. By way of example, the DIASTAT (TM) Anti-CCP ELISA assay available from Axis-Shield Diagnostics, Dundee, UK is a commercially and widely available heterogeneous assay method for anti-CCP, which provides an assay result in arbitrary units per ml, termed U/ml. Evidently, the method of the present invention may be calibrated against such a method by running a series of samples using both methods and constructing a calibration curve to relate the two scales. This then allows conversion of the threshold and reference values from the known platform to the new homogeneous method of the invention.

Where the method of the invention has been calibrated against the above DIASTAT (TM) Anti-CCP ELISA assay, the equivalent reference range within which the value for anti-CCP concentration will generally be found (for a healthy individual) is <1.0 to 2.9 U/mL. An anti-CCP concentration in serum or plasma of greater than 5.0 U/mL (e.g. 3.5 to 2000, preferably 5.0 to 500, especially 8.0 to 200 (or greater) U/ml) will generally be very strongly indicative of potential for RA or propensity to RA. In one embodiment, the higher the anti-CCP value above a threshold (such as 3 U/ml, preferably 5 U/ml) the higher will be considered the tendency to RA.

A particularly preferred assay method for assessing the concentration of anti-CCP in the present invention is a particle-based immunoassay. This is a sensitive technique which is based on turbidimetric detection of the aggregation of nano-particles in suspension mediated by the anti-CCP antibody, and correspondingly determination of the anti-CCP concentration. The sensitivity provided by the assay advantageously allows for the relatively low concentrations of anti-CCP in body fluid (e. g. plasma or serum) samples to be determined with a high level of precision. At the same time, relatively high concentrations of anti-CCP can also be measured with accuracy.

Typically, calibration samples having anti-CCP contents over a broad range will be used to calibrate and standardise the assay method. The most effective and representative calibration samples are generally those provided from clinical sample taken from a patient with very high anti-CCP antibody count, diluted as necessary to form a calibration range. A calibration range of 0 to 2000 U/mL standardised against the above DIASTAT (TM) Anti-CCP ELISA assay will be highly appropriate, although in practice the range 0 to 200 U/mL may be sufficient to provide the necessary clinical information. No international reference standard currents exists for this analyte therefore, the assay uses arbitrary Axis-Shield units (U/mL) as described in detail herein. It is preferable that the assay method of the invention is sensitive to CCP down to a concentration of 0.5 U/ml, preferably down to 0.1 U/ml. Thus, the detectable range should be around 0.1 to 2000 U/ml, e.g. 0.1 to 200 U/ml.

As indicated above, it will be advantageous to calibrate the methods of the present invention against standard samples directly, and appropriate thresholds should be determined by testing of patient groups and control groups of healthy individuals. In many cases, it is not necessary to know what absolute concentration of anti-CCP antibody correlates to a particular threshold or detected value. An arbitrary scale is sufficient providing that it is reproducible, and preferably that it can be related to the scales used in other common methods, such as the DIASTAT method discussed above, to allow direct comparison of results. The assay methods of the invention in all aspects may be qualitative, semi-quantitative or fully quantitative, but a qualitative assay wherein the level is assessed against one or more control samples is the embodiment most easily practiced. By suitable methods, such as interpolation into a standard curve, however, semi-quantitative or fully quantitative assays on an arbitrary or absolute scale may be practiced.

The above scale of 0.1 to 200 U/ml is believed to correspond to approximately 10ng/ml to 20 µg/ml of anti-CCP antibody however, this is dependent on the variation in affinity of different antibodies. Thus, although it is both preferable and to some extent necessary to determine one or more threshold values by reference to populations of subjects with and without RA, for human subjects it is possible to use this as a guide to the appropriate detection range and threshold values. Thus, a suitable threshold for the presence or absence of RA may be around 0.1 µg/ml to around 5µg/ml, particularly around 0.2 µg/ml to around 2 µg/ml, most preferably around 0.4µg/ml to around 1µg/ml. Correspondingly, appropriate detection ranges and suitable ranges for standard solutions or calibration solutions are around 1 ng/ml to around 200µg/ml, preferably 10ng/ml to around 100µg/ml.

Since turbidimetric methods are a highly preferred embodiment of the present invention and the equipment required for automating such assays is well known, as discussed above, the invention will be described primarily by reference to turbidimetric methods. It should be appreciated, however, that any assay method based upon bringing components together to generate a new signal, such as those described herein above, may be used equivalently by adapting the methods disclosed below using labelling/detection systems conventional in the art. Since a turbid solution may be analysed by absorption (turbidimetric) or scattering (nephelometric) methods, the term "turbidimetry" as used herein includes nephelometric detection where context allows.

Turbidimetric determination in the present invention is homogeneous and thus also has the advantage that no solid surface is required for physical separation in the assay and numerous washing and/or separation steps are not required. Thus compared to prior art techniques (e. g. ELISA), in the homogenous methods of the invention, determination of anti-CCP is quick and easy to perform and may, for instance, be readily automated.

As discussed above, an automated, homogeneous turbidimetric assay is also fast, allowing for a high throughput of samples, and is relatively cheap to run. Typically it can be performed using a commercially available robot, e. g. the Cobas Mira or Hitachi 711, both of which are available from Roche Diagnostics. Such an automated assay is particularly attractive when routine testing of individuals (such as those from high-risk groups as described herein) for potential for or propensity to RA is envisaged.

In all methods of the present invention, a key step is determination of anti-CCP concentration, and the anti-CCP-containing sample will generally be a body fluid, e.g. urine, cerebrospinal fluid, oral fluid, synovial fluid or emphysema fluid, or more preferably, whole blood or a blood derived sample. When a blood derived sample is used, the sample used for analysis will preferably be cell-depleted (e. g. serum or plasma). Cell depletion will typically be by removal of at least 70% of the cells from a sample, preferably at least 80% and more preferably at least 90%. Up to substantially 100% of the cells may be removed. Any of the sample fluids, including blood and blood derivatives may be treated to remove any cells and/or any sample components not being assayed for. The sample may also be treated to concentrate (e.g. by transfer of the anti-CCP antibodies to a separate (e. g. solid substrate) medium, separation and re-suspension at higher concentration) or dilute the sample. Other well-known methods of sample pre-treatment, including centrifugation, separation of specific components, or addition of preservatives such as anticoagulants is also appropriate if necessary. For instance, the sample may be diluted by adding water, a buffer or other aqueous medium. Alternatively and preferably, a sample, particularly a whole-blood, serum or plasma sample, may be used directly. The sample used in the methods of the invention is generally an "anti-CCP containing sample", which is used herein to indicate a sample typically containing anti-CCP, especially in a subject having or having propensity to RA. Thus, the presence of anti-CCP in such a sample is assumed at some level but determined by the assay method. Correspondingly, the absence of anti-CCP in such a sample at an appropriate detection level can be taken to indicate a lack of RA, or a lowered risk of or propensity to RA.

The subject from whom the sample is taken and for whom the diagnosis or analysis of risk or probability will be made is a human or non-human animal subject, especially a mammal and preferably a human, canine or feline mammal. Human subjects are most preferred. The subject may be a subject with or without any existing clinical manifestations of RA. Any subject is suitable for testing, but most suitable are individuals having some known risk or indication of RA, and those in at-risk groups, such as older subjects (e.g. human subjects over 50 years of age, especially over 60 and most especially over 65 years of age, such as subjects between 50 and 100), subjects having suffered bone injuries or damage, or subjects whose joints have suffered increased or abnormal joint wear, such as athletes, or those having or having had occupations predisposing them to joint wear. Subjects having other conditions pre-disposing them to RA are also highly suitable, especially those having inflammatory and/or autoimmune diseases, those having a family history of RA and/or those for whom genetic testing has indicated a predisposition towards developing RA. Other potential groups of subjects include those with clinical symptoms which might indicate RA, but could indicate other conditions, such as joint stiffness or joint pain. The method is particularly beneficial in patients having no symptoms (such as outwardly healthy subjects in high-risk groups), or having only minor or early symptoms of RA, so that a diagnosis may be made, confirmed or supported and appropriate treatment begun before irreversible damage occurs.

One key aspect of the present invention relates to the use of at least one CCP peptide antigen as specific binder for the anti-CCP antibody. The synthesized CCP peptide is known and available using common techniques or commercial peptide synthesis. One could also make a recombinant peptide and modify if necessary to produce CCP peptide. Sequences and descriptions of suitable CCP peptides and analogues thereof are available in WO98/22503. Suitable CCP peptides maybe bound, coupled or linked (directly or indirectly) to a signal generating moiety and thus used to generate a signal corresponding to the degree of CCP/anti-CCP binding. Any CCP peptide obtained, for example, by any conventional technique for making peptides, may be used in the method of the invention.

Anti-CCP antibodies have been shown react specifically with linear peptides of appropriate sequence, as well as with cyclic citrullated peptides and their cyclic analogues (see, for example WO98/22503). CCP peptides for use in the present invention may thus be cyclic, but whether cyclic or linear, they will be peptides having at least one modified arginine residue, especially arginine modified to be citrulline, or an analogue thereof. Such peptides may be cyclic due to the presence of a disulphide bond within the chain, although other forms of cross-linking may be used. Suitable peptides may be of any size, but preferred peptides will be of around 5 to 200 residues in length, preferably 8 to 80 residues, and more preferably around 10 to 30 residues in length. Particular reference is made to the formula sheet of the above-mentioned WO98/22503, and to claim 2 thereof, which together contain definitions of particularly preferred citrulline moieties and of particularly preferred CCP peptide sequences.

The signal generating moiety is a particle in the case of a turbidimetric assay. As the signal generating moieties are brought together by the act of binding, and particularly dual binding/cross-linking, by the anti-CCP antibodies in the sample, a signal is generated and may be detected.

In a turbidimetric assay several signal binding moieties may be brought together to form an extended network. This may be achieved by binding more than one (e.g. 2 to 1000, especially 2 to 100) specific binder to a single signal generating moiety. In one embodiment of the present invention, the signal generating moiety may be the specific binder itself rather than a separate entity. In such cases, it is the act of binding which generates the signal, and such signals are typically physical changes, such as a difference in the scattering properties of the sample, or very small changes in sample temperature due to liberated binding energy. Where the specific binder is inherently multi-valent (e.g. divalent) such as an antibody, and in particular if there are a variety of such binders recognising differing regions (epitopes) of the anti-CCP-antibody (e.g. at least two different anti-CCP binding aptamers recognising different regions), then the mass of the binder itself may be the signal generating moiety, because when the specific binders are brought together, a cross-linked network will form generating aggregates of detectable size. Similarly, where several CCP antigenic sequences are linked together, either in a single peptide or as antigens attached to a backbone molecule or particle, a cross-linked network will form generating aggregates of detectable size.

In a turbidimetric assay, opacity may be generated by contacting the anti-CCP-containing sample, or an aliquot thereof, with a CCP peptide antigen, preferably bound to an opacity generating moiety, such as a nano-particle. In a particularly preferred method, two or more (e.g. 2 to 1000, especially 2 to 100) anti-CCP peptides may be bound to each particle.

CCP peptides useful in the methods of the invention for determination of anti-CCP concentration preferably show no or little cross reactions with other blood proteins that may be present in the eluate. The quantity of peptide used in any case can of course be optimized against anti-CCP-containing standard samples. This is particularly important in methods such as turbidimetry and other methods involving cross-linking/oligomerisation as these effects may be non-linear. For turbidimetry, opacification arises from the hook effect whereby multiple anti-CCP binding generates the opacification centres. The required degree of binding for this effect will, however, depend upon factors such as the size of the signal generating moieties and the average number of specific binders linked to each of these.

In one preferred turbidimetric embodiment, CCP peptide, may be immobilized by binding or coupling, either directly or indirectly, to any well known solid support or matrix which is commonly used for immobilization in homogeneous turbidimetric assays.

In all turbidimetric methods of the invention (including those using nephelometric detection), the solid support or matrix preferably takes the form of particles, most preferably particles which may be suspended in aqueous media and are smaller than the wavelengths of red, preferably blue and more preferably ultra-violet light (e.g less than 250 nm). Nanoparticles are most preferred, especially those having a diameter of between 10 and 250 nm. Conveniently the solid support may be made of metal (e. g. gold) or a polymeric material (e. g. polyethylene). Preferably the solid support is made of a polymeric material such as polyethylene.

The particles to which the specific binder (e.g. CCP peptide, antibody, antibody fragment or aptamer), may be bound in a turbidimetric embodiment are typically spherical. The size of the particles used in the assay may effect the precision with which the anti-CCP concentration is measured. Whilst larger particles allow for lower concentrations of anti-CCP to be detected, their reduced surface area means that they have a lower binding capacity. For example, doubling the particle diameter, halves the binding capacity of a mass unit of particles.

Additionally increasing the particle diameter increases the level of background light absorbance and light suspension at the wavelengths typically used in such assays (e.g. 330 to 600 nm). Thus whilst larger particles increase the sensitivity of the assay, this may be accompanied by some loss of accuracy and in particular, an increase in the number of false negative results obtained. This is particularly likely to be the case with samples containing relatively high anti-CCP concentrations (i.e. those samples obtained from individuals with a high potential for or propensity to RA) wherein the nanoparticle- bound binding sites may become saturated without all of the anti-CCP becoming bound. By appropriate design of the assay, however, it can be ensured that this saturation point is reached at a concentration well above the relevant threshold for clinical diagnosis. If it is necessary to quantify this very high level then the assay operator can be prompted to carry out a secondary test, either using alternative reagents suitable for a higher anti-CCP concentration range, or more preferably simply by diluting the original sample by a known factor (e.g. 10 or 100) and re-submitting it in the original assay method.

These counter-acting effects associated with changing the particle size (e. g. increasing the particle size increases sensitivity but decreases accuracy) represent a significant problem to be overcome in the development of a sensitive assay for detecting the range of levels of anti-CCP present in body fluids by turbidimetry. Such assays are described herein, however, and illustrated in the accompanying examples.

It is further preferable in the assay method of the invention that the particles and method used allow for a wide range of anti-CCP concentrations to be determined with precision. This may mean that a high level of confidence can be attributed to both a negative result (i.e. a concentration falling below the threshold value) as well as a positive result. It is particularly preferred in the assay method of the invention that samples having an anti-CCP concentration in the range 1pg/ml to 100 µg/ml, preferably 1ng/ml to 50 ug.ml, more preferably 10ng/ml to 10µ/ml, (e.g. 10 ng/ml to 1µg/ml or 100ng/ml to 10µg/ml, corresponding approximately to, for example, 0.05 U/mL to 200 U/mL, or 0.5 to 200 U/mL) can be measured.

The particles, in both the "nude" and coated states, preferably have a diameter which does not itself enable absorption of the wavelength of light used for spectrophotometric determination. Thus the suspension of coated nanoparticles is approximately (e.g. substantially) transparent until anti-CCP induced aggregate formation occurs resulting in the formation of aggregates having a larger diameter. Such aggregates have the ability to absorb and scatter the wavelength of light used by the spectrophotometer.

Further, the particles are preferably substantially all of the same size, more specifically all of the same diameter. It may be, therefore, that less than 1% of the particles have a diameter of more than twice the average. Preferably, monodisperse metal (e. g. gold) or polymer particles are used. Monodisperse polymer particles are available from Dynal Biotech AS, Oslo, Norway.

Whilst not wishing to be bound by theory, it is believed that by using a solid support or matrix (e. g. nanoparticles) which is substantially all of the same size it may be that the sensitivity of the turbidimetry assay is increased.

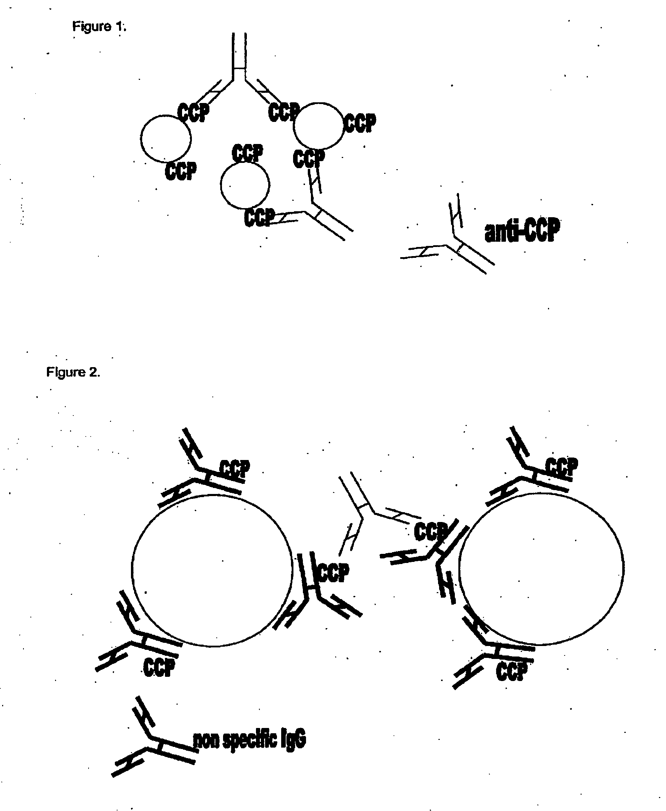

Binding or immobilization of the CCP peptide or other peptide specific binders may be achieved using any conventional technique. For example, avidin (available from Pierce Chemical Company) may be immobilized on chloromethyl activated polystyrene nanoparticles (available from Interfacial Dynamic Corporation, US) by agitation in buffer (e. g. at room temperature for 24 hours) and then used in conjunction with biotin labelled CCP peptide (prepared according to conventional techniques in the art). Thus, for example, plasma taken from the subject to be tested is added to a solution of avidin-coated nanoparticles in a quartz cuvette of a spectrophotometer, followed by the addition of biotin labelled CCP peptide. Turbidimetric readings are then taken. Results of such a method are illustrated in Figure 1.

Alternatively, the biotin labelled peptide may be added prior to the addition of plasma or serum. In other words, whilst the same reagents are typically used regardless of the instrument used for turbidity detection, the precise sequence in which the various reagents are added may vary. Generally, the sequence used should be in accordance with the instructions accompanying the spectrophotometer used (e.g. a Shimadzu UV-160 spectrophotometer).

Turbidimetric readings are made (i.e. the light absorption or light scattering at a suitable wavelength is measured at regular intervals) and the measured value relative to a reference is determined. Analogous methods are used for other detection methods, as is known in the art. Optionally, multiple wavelength instruments may be used to make turbidimetric readings and may provide more precise results. Suitable instruments for taking turbidimetric readings include the Cobas Mira, Roche Integra and Merck's Turbiquant.

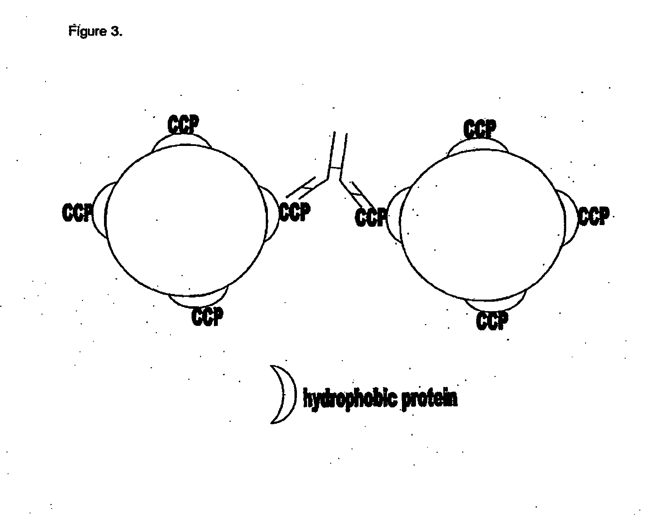

In an alternative experimental set-up, binding or immobilization of the CCP peptide to a particulate opacity generating moiety may be achieved using any other conventional technique. For example, non specific IgG or hydrophobic proteins may be immobilised on chloromethyl activated polystyrene nanoparticles (available from Interfacial Dynamic Corporation, US) by agitation in buffer (e.g. at room temperature for 24 hours) and coupled to CCP peptide using cross-linking reagents (available from Pierce, US, prepared according to conventional techniques in the art) prior to or after binding of the IgG molecules to the nanoparticles. Thus, for example, plasma taken from the subject to be tested for potential for, or propensity to, RA is added to a solution of coated nanoparticles in a quartz cuvette of a spectrophotometer. Turbidimetric readings are then taken. See figures 2 and 3.

The same reagents are typically used regardless of the instrument used for turbidity detection, the precise sequence in which the various reagents are added may vary. Generally, the sequence used should be in accordance with the instructions accompanying the spectrophotometer used (e. g. a Shimadzu UV-160 spectrophotometer).

Examples of automated robots which are suitable for taking turbidimetric readings in accordance with the turbidity assay methods of the invention include the Cobas Mira and Hitachi 711, both of which are available from Roche Diagnostics.

By using a solid support or matrix (e. g. nanoparticles) which is substantially all of the same size it may be that the sensitivity of the turbidimetry assay is further increased, as indicated above.

Various features of the assay methods of the invention may be optimised to improve the results. These include the following preferable features, which are all optional and usable independently.
Optionally, in determining anti-CCP concentration, a kinetic reading mode may be used. This method may preferably be used in all aspects of the invention, particularly the turbidimetry technique.
In general, in addition to the sample under evaluation, calibration samples with known anti-CCP contents will also be assessed in the performance of the assay method. Such determinations can be used to plot a calibration curve from which the anti-CCP content of the sample under evaluation can be determined.
Preferably calibration samples having anti-CCP contents of up to 10 µg/ml (e.g. any selection of 0, 1, 2, 4, 5, 8 and 10 µg/ml, or any others between) will be used.
Where stimulation of a signal (e.g. of a fluorophore for FRET or FP) is required, this stimulation will typically be by electromagnetic radiation, especially by light, such as monochromatic light, including laser light. Appropriate wavelengths will be, for example, 100-800 nm, preferably 250 to 600 nm, and most preferably 300 to 600 nm. Detection may be (independently in the case of FRET) in similar ranges.

In the preferred turbidimetric detection method, several additional features may be optimised for best performance of the assay method:
As is routine in turbidimetric assays, a polymeric opacification enhancer, such as polyethyleneglycol, is preferably also added to the eluate.
Before the turbidimetric determination is made, the fraction, antibody or antibody fragment, preferably bound to a nanoparticle, and optionally including an opacification enhancer, may be incubated for a short period, e.g. 5 minutes to an hour, preferably 8 to 20 (e.g. about 10) minutes, at room temperature.
The light used in the determination of opacification should have an appropriate wavelength, for example, 300-600 nm. In this regard it was found that use of a 300-450 nm filter, preferably a 340 nm or a 405 nm filter, furnished particularly good results. Use of a 560 nm filter may also yield especially good results.

The above described assay method for the determination of anti-CCP (especially by turbidimetric methods) is surprisingly reliable, quick, cheap, facile and amenable to automation. This is in contrast to the currently available assay methods which are relatively complex and are not directly applicable to the automated multi-task diagnostic machines commonly used by diagnostic laboratories.

Automation is particularly desirable where numerous mixing, addition and/or dilution steps are involved since these may be achieved with a higher degree of accuracy. Robots may also offer a higher level of reliability and/or reproducibility. Automation also increases throughput.

The currently available assay methods which may offer reasonable levels of precision (e. g. ELISA) are, however, difficult to automate. This is at least in part because they typically involve numerous washing and separation steps (e. g. attachment to a solid surface) and automation of non-homogeneous processes is often problematic. Also these processes typically involve a relatively large number of steps which increases the complexity of any automated protocol.

Thus, according to a highly preferred embodiment, the present invention provides an assay method for the determination of anti-CCP in an anti-CCP-containing body fluid, said method comprising the steps of: (a) obtaining an anti-CCP-containing liquid sample of, or derived from, said fluid; (b) contacting said sample of said body fluid with an, optionally nanoparticle-bound, CCP peptide, to bind said anti-CCP; (c) optionally, adding an opacity enhancer; and (d) assessing the anti-CCP content by turbidimetry.

Such an assay may be useful in the diagnosis of RA condition which is characterized by abnormal levels (e. g. high levels) of anti-CCP. Thus elevated anti-CCP, such as that which is at a measurable level or is above a pre-determined threshold value such as those indicated herein, may be taken as a risk factor in the development of RA in any population including any individual or combination of those populations indicated herein. The invention thus provides a corresponding method for assessing the risk of RA in a subject, including the risk of existing RA, the risk of potential RA, and/or the risk of propensity to RA. Elevated anti-CCP may be used as the sole risk factor, or may be used in combination with other risk factors. Evaluation of anti-CCP according to the present invention may also be used in verifying or supporting the existence of RA in a subject having possible symptoms of RA, or being at risk of RA. Suitable populations include all of those mentioned herein in any combination.

A body sample used in the turbidimetric assay method may be any anti-CCP-containing sample, or any sample potentially containing anti-CCP antibody, e. g. a body fluid or tissue sample, or a suspension etc. Preferable sample types are described herein above.

In one aspect, the invention provides a kit for performing the assay methods according to the invention, said kit comprising at least one specific binder for anti-CCP immobilised to monodisperse metal or polymer particles having a diameter of between 10 and 250 nm wherein the binder for anti-CCP is at least one CCP peptide antigen and wherein two or more such peptides are bound to each particle. The kit preferably also comprises at least one signal generating moiety, preferably, an anti-CCP solution of known concentration and more preferably a set of such solutions having a range of anti-CCP concentrations; preferably, at least one opacity enhancer, a light transmitting vessel; and preferably, a detector.

The present invention will now be described with reference to the following examples which are provided for the purpose of illustration and are not intended to be construed as being limited on the present invention. The invention is additionally illustrated by the attached figures in which:
Figure 1 Schematically represents cross-linking by anti-CCP in a solution of avidin-coated nanoparticles and biotin labelled CCP peptide;
Figure 2 Schematically represents cross-linking by anti-CCP in a solution of nanoparticles coated with secondary antibodies with CCP bound thereto; and
Figure 3 Schematically represents cross-linking by anti-CCP in a solution of nanoparticles coated with hydrophobic proteins having CCP bound thereto.

### Example 1

Turbidimetric Assay for Anti-CCP (a) Preparation of Avidin-coated Nanoparticles 600 µl of 4.2% w/v chloromethyl activated nanoparticles (diameter 44 nm) available from Interfacial Dynamic Corporation, US are dialyzed against water with a membrane having a pore size of 300,000 Da.

0.5 ml of a borate (10 mM) and sodium chloride (15 mM) solution at pH 9.0 is added and mixed. 10 mg avidin, dissolved in 0.5 ml of a 10 mM borate and 15 mM NaCl solution at pH 9 (available from Pierce Chemical Company) is added and the mixture is agitated at room temperature for 24 hours. 40 µl of glycine solution (2M, pH 9.0) is then added and the mixture is agitated for a further 4 hours at room temperature.

The particles are then diluted to a volume of 100 ml and diafiltrated, firstly in 500 ml of a 10 mM borate and 15 mM sodium chloride solution at pH 9.0 and secondly in a 25 mM Tris, 150 mM sodium chloride and 0.01 % Tweens 20 solution at pH 7.4 (available from Sigma US) using a Pellicon XL Filter (cut off 300,000) and a labscale TTF System (available from Millipore) in accordance with the instructions supplied from the instruments suppliers. The desired concentration of avidin-coated nanoparticles is finally obtained by centrifugation and re-suspension of the particles in a 25 mM TRIS, 150 mM sodium chloride and 0.01% Tween 20 solution. Any aggregates formed during this preparation procedure may be removed by slow centrifugation.

### (b) Assay for Anti-CCP using Avidin-coated Nanoparticles

A suspension having a concentration of about 0.30 mg particles of the above-described avidin-coated nanoparticles per ml is prepared by centrifugation and re-suspension of the above-described preparation in a 25 mM TRIS, 150 mM NaCl, 0.1 % Tween 20 and 2 % PEG 6000 solution at pH 7.4 (available from Sigma). 500 µl of this particle suspension is mixed with a plasma sample (about 20 µl), taken from a subject being tested for propensity to RA, in a reading quartz cuvette of a recording spectrophotometer (e. g. a Shimadzu UV-160).

The absorption of 340 nm monochromatic light is recorded and after 60s, 1.5µg of CCP peptide labelled with 1.0 nmol biotin (e. g. biotin labelled CCP peptide), diluted in 50 µl of a 25 mM TRIS, 150 mM NaCl and 0.1% Tweens 20 solution at pH 7.4 is added to the quartz cuvette and mixed. The absorption of 340 nm monochromatic light is immediately recorded using a reference cuvette containing a solution of 25 mM TRIS, 150 mM NaCl and 0.1 % Tween 20 at pH 7.4, and again at regular intervals (e. g. every 2 minutes) until about 15 minutes has elapsed. The increase in absorption at each time point is calculated in accordance with standard turbidimetric reading in kinetic mode or "end-point" readings. That is, the increase in light absorption at each time-point is calculated relative to the reading made prior to the addition of CCP-coated nanoparticles and/or at the end of the recording.

A calibration curve is constructed by carrying out an identical procedure with standards having a known concentration of anti-CCP. The concentration of anti-CCP in the sample is then calculated from the calibration curve.

Example 2: Alternative Turbidimetric Assay for Anti-CCP (a) Preparation of CCP peptide Coated Nanoparticles 1 ml of 4.2% w/v chloromethyl activated nanoparticles (diameter 44 nm) available from Interfacial Dynamic Corporation, US are dialysed against water with a membrane having a pore size of 300,000 Da.

0.5 ml of a 10 mM borate and 15 mM sodium chloride solution at pH 9.0 is then added. 1.5µg of purified CCP peptide (e. g. affinity purified CCP peptide) is dialysed against a 10 mM borate and 15 mM sodium chloride solution at pH 9.0.

Following addition of the nanoparticles to the purified CCP peptide the mixture is agitated for 24 hours at room temperature. 40 µl of a glycine solution (2 M at pH 9.0) is then added and the mixture is agitated for a further 4 hours at room temperature.

The particles are then diluted to total volume of 100 ml and diafiltrated against 1000 ml of a 10 mM borate and 15 mM sodium chloride solution at pH 9.0 to which 0.10% Tween 20 and 3 mg/ml egg albumin is added using a Pellicon XL filter (cut of 300,000) and a labscale TFF system (available from Millipore) in accordance with the instructions supplied from the instruments suppliers.

The desired concentration of CCP peptide- coated nanoparticles is finally obtained by centrifugation and re-suspension of the particles in solution. Any aggregates formed during this preparation procedure are optionally removed by slow centrifugation.

### (b) Assay for Anti-CCP using CCP peptide-coated Nanoparticles

A suspension comprising 400 µg of the above- described CCP peptide-coated nanoparticles in 50 µl of a 10 mM borate, 15 mM NaCl, 0.1% Tween 20, 3g/l egg albumin solution at pH 9.0 is prepared.

Simultaneously, 20 µl of plasma, taken from the subject being tested for potential for RA, in 500 µl assay buffer (25 mM TRIS, 150 mM NaCl, 0.1 % Tweens 20 and 2% PEG 6000 at pH 7.4 (available from Sigma)) is put in a reading quartz cuvette of a recording spectrophotometer (e. g. ShimadzuW-160) and the light absorption of 340 nm monochromatic light is measured.

After 60s, the above-mentioned suspension comprising 400µg of CCP peptide-coated nanoparticles is added, and mixed in the cuvette. The light absorption immediately after adding the CCP- coated nanoparticles is recorded, and again at regular intervals (e. g. every 2 minutes) until about 15 minutes has elapsed. The increase in light absorption at each time-point is calculated relative to the reading made prior to the addition of CCP-coated nanoparticles and/or at the end of the recording. In other words, turbidimetric readings in kinetic mode or "end-point" readings are made.

A calibration curve is also constructed by carrying out an identical procedure with standards having a known concentration of anti-CCP. The concentration of anti-CCP in the sample is then calculated from the curve.

### Example 3: Turbidimetric Assay for Anti-CCP (a) Preparation of Streptavidin-coated Nanoparticles

600 µl of 4.2% w/v chloromethyl activated nanoparticles (diameter 67 nm) available from Interfacial Dynamic Corporation, US are dialysed against water with a membrane having a pore size of 300,000 Da.

0.5 ml of a phosphate (10 mM) and sodium chloride(150 mM) buffer solution at pH 7.4 is added together with 10 mg streptavidin, dissolved in 0.5 ml of a 10 mM phosphate and 150 mM NaCl buffer solution at pH 7.4 (available from Pierce Chemical Company) and the mixture is agitated at room temperature for 24 hours. 40 µl of glycine solution (2M, pH 9.0) is then added and the mixture is agitated for a further 4 hours at room temperature.

The particles are then diluted to a volume of 100 ml and diafiltrated, firstly in 500 ml of a 10 mM borate and 15 mM sodium chloride solution at pH 9.0 and secondly in a 25 mM Tris, 150 mM sodium chloride and 0.01% Tween 20 solution at pH 7.4 (available from Sigma US) using a Pellicon XL Filter (cut off 300,000) and a labscale TTF System (available from Millipore) in accordance with the instructions supplied from the instruments suppliers. The desired concentration of Streptavidin-coated nanoparticles is finally obtained by centrifugation and re-suspension of the particles in a 25 mM TRIS, 150 mM sodium chloride and 0.01% Tween 20 solution. Any aggregates formed during this preparation procedure may be removed by slow centrifugation.

The mean particle size of the streptavidin coated nanoparticles is measured to be 82 nm.

### (b) Assay for Anti-CCP using Streptavidin-coated Nanoparticles

A suspension having a concentration of about 0.60 mg particles of the above-described Streptavidin-coated nanoparticles per ml is prepared by centrifugation and re-suspension of the above-described preparation in a 25 mM TRIS, 150 mM NaCl, 0.1% Tween 20 and 1% PEG 6000 solution at pH 7.4 (available from Sigma). 500 µl of this particle suspension is mixed with a plasma sample (about 5 µl), taken from a subject being tested for propensity to RA, in a reading quartz cuvette of a recording spectrophotometer (e. g. a ShimadzuW-160).

The absorption of 560 nm monochromatic light is recorded and after 60s, 1.5 µg of CCP peptide labelled with 1.0 nmol biotin (e. g. biotin labelled CCP peptide), diluted in 50 µl of a 25 mM TRIS, 150 mM NaCl and 0.1% Tween 20 solution at pH 7.4 is added to the quartz cuvette and mixed. The absorption of 340 nm monochromatic light is immediately recorded using a reference cuvette containing a solution of 25 mM TRIS, 150 mM NaCl and 0.1 % Tween 20 at pH 7.4, and again at regular intervals (e. g. every 2 minutes) until about 15 minutes has elapsed. The increase in absorption at each time point is calculated in accordance with standard turbidimetric reading in kinetic mode or "end-point" readings. That is, the increase in light absorption at each time-point is calculated relative to the reading made prior to the addition of CCP- coated nanoparticles and/or at the end of the recording.

A calibration curve is constructed by carrying out an identical procedure with standards having a known concentration of anti-CCP. The concentration of anti-CCP in the sample is then calculated from the calibration curve.

### Example 4: Turbidimetric Assay for Anti-CCP (a) Preparation of hydrophobic protein- coated Nanoparticles

600 µl of 4.2% w/v chloromethyl activated nanoparticles (diameter 67 nm) available from Interfacial Dynamic Corporation, US are dialysed against water with a membrane having a pore size of 300,000 Da.

0.5 ml of a phosphate (10 mM) and sodium chloride (150 mM) buffer solution at pH 7.4 is added together with 10 mg hydrophobic protein (e. g. bovine serum albumin), dissolved in 0.5 ml of a 10 mM phosphate and 150 mM NaCl buffer solution at pH 7.4 (available from Pierce Chemical Company) and the mixture is agitated at room temperature for 24 hours. 40 µl of glycine solution (2M, pH 9.0) is then added and the mixture is agitated for a further 4 hours at room temperature.

The particles are then diluted to a volume of 100 ml and diafiltrated, firstly in 500 ml of a 10 mM borate and 15 mM sodium chloride solution at pH 9. 0 and secondly in a 25 mM Tris, 150 mM sodium chloride and 0.01 % Tween 20 solution at pH 7.4 (available from Sigma US) using a Pellicon XL Filter (cut off 300,000) and a labscale TTF System (available from Millipore) in accordance with the instructions supplied from the instruments suppliers. The desired concentration of hydrophobic protein- coated nanoparticles is finally obtained by centrifugation and re-suspension of the particles in a 25 mM TRIS, 150 mM sodium chloride and 0.01% Tween 20 solution. Any aggregates formed during this preparation procedure are optionally removed by slow centrifugation.

The mean particle size of the hydrophobic protein- coated nanoparticles is measured to be 82 nm.

CCP peptide is coupled to hydrophobic protein- nanoparticles using cross-linking reagents (available from Pierce, US) prepared according to conventional techniques.

### (b) Assay for Anti-CCP using hydrophobic protein-coated Nanoparticles

A suspension having a concentration of about 0.60 mg particles of the above-described hydrophobic protein- coated nanoparticles per ml is prepared by centrifugation and re-suspension of the above-described preparation in a 25 mM TRIS, 150 mM NaCl, 0.1 % Tween 20 and 1% PEG 6000 solution at pH 7.4 (available from Sigma). 500 µl of this particle suspension is mixed with a plasma sample (about 5 µl), taken from a subject being tested for propensity to RA, in a reading quartz cuvette of a recording spectrophotometer (e. g. a ShimadzuW-160). The absorption of 340 nm monochromatic light is immediately recorded using a reference cuvette containing a solution of 25 mM TRIS, 150 mM NaCl and 0.1 % Tween 20 at pH 7. 4, and again at regular intervals (e. g. every 2 minutes) until about 15 minutes has elapsed. The increase in absorption at each time point is calculated in accordance with standard turbidimetric reading in kinetic mode or "end-point" readings. That is, the increase in light absorption at each time-point is calculated relative to the reading made prior to the addition of CCP-coated nanoparticles and/or at the end of the recording.

A calibration curve is constructed by carrying out an identical procedure with standards having a known concentration of anti-CCP. The concentration of anti-CCP in the sample is then calculated from the calibration curve.

## Claims

1. A method for assaying anti-cyclic citrullinated peptides (anti-CCP) antibodies in a clinical sample selected from blood, blood derivatives, serum, plasma, urine, cerebrospinal fluid, oral fluid, synovial fluid and emphysema fluid, said method comprising providing a homogenous assay format, contacting said sample with at least one reagent comprising at least one specific binder for anti-CCP antibodies immobilised to monodisperse metal or polymer nanoparticles having a diameter of between 10 and 250 nm, whereby to form a solution or suspension of an anti-CCP-binding partner complex in a homogeneous sample mixture and detecting the presence or level of said anti-CCP-binding partner complex in a homogeneous liquid-phase, wherein said detection is carried out by turbidimetry, wherein in said method the sample and at least one reagent are mixed, a signal generated and that signal detected without any separation or washing steps involving phase separation and wherein the binder for anti-CCP is at least one CCP peptide antigen where two or more such peptides are bound to each particle.

2. A method for the assessment of the existence of; risk of; potential for; or propensity to Rheumatoid Arthritis (RA) in a subject, said method comprising assaying for anti-CCP in a body sample from said subject in a homogeneous assay according to claim 1, whereby to determine the level of anti-CCP antibodies in said sample, and correlating the thus-determined level with the existence of; risk of; potential for; or propensity to RA in said subject wherein the existence of anti-CCP, or a concentration of anti-cyclic citrullinated peptides (anti-CCP) above one or more threshold values is correlated with existence of, increased severity of, increased risk of, increased potential for, and/or increased propensity to RA, and non-existence of anti-CCP or a concentration of anti-CCP below one or more threshold values is correlated with non-existence of, decreased severity of, decreased risk of, decreased potential for, and/or decreased propensity to RA, wherein said threshold value is such that 80% of patients with clinically confirmed RA will provide samples geneating a signal above that value and wherein a concentration of anti-CCP above said threshold values is correlated with existence of, increased risk of, increased potential for, and/or increased propensity to RA.

3. A method as claimed in claim 2 including the assessment of other biochemical markers, such as Rheumatoid Factor (RF), in combination with the assessment of anti-CCP.

4. A method as claimed in any of claims 1 to 3 wherein said specific binder for anti-CCP antibodies is bound to at least one signal generating moiety, preferably wherein a detectable signal is generated by the formation of a complex containing said signal generating moiety and at least one other signal generating moiety of the same or different type.

5. A method as claimed in claim 4 wherein said signal generating moiety is a nanoparticle, preferably wherein said nanoparticle is bound to more than one specific binder for anti-CCP antibody.

6. A method as claimed in any of claims 1 to 5, said method additionally comprising the step of adding an opacity enhancer.

7. A method as claimed in any of claims 1 to 6 further comprising performing the method on calibration samples having anti-CCP contents of 0 to 20 µg/ml of anti-CCP antibody.

8. A method as claimed in any of claims 2 to 7 wherein said subject is a subject selected from the group of subjects consisting of older subjects, subjects having suffered bone injuries or damage, subjects whose joints have suffered increased or abnormal joint wear, subjects having inflammatory and/or autoimmune diseases, subjects having family history of RA, subjects having had a genetic test indicating increased propensity to RA, and subjects having clinical symptoms which equivocally indicate RA.

9. A kit for performing the methods as claimed in any of claims 1 to 8, comprising at least one specific binder for anti-CCP antibody immobilised to monodisperse metal or polymer nanoparticles having a diameter of between 10 and 250 nm wherein the binder for anti-CCP is at least one CCP peptide antigen and wherein two or more such peptides are bound to each particle.

10. A kit as claimed in claim 9 where the binding of the homogeneous specific binder and anti-CCP antibody react to generate a signal.

11. A kit as claimed in claim 10 further comprising at least one of the following constituents;
at least one signal generating moiety;
at least anti-CCP solution of known concentration;
at least one opacity enhancer;
a light transmitting vessel;
a detector.

## Patentansprüche

1. Verfahren zum Analysieren von Antikörpern gegen antizyklisches citrulliniertes Peptid (Anti-CCP-Antikörpern) in einer klinischen Probe, ausgewählt aus Blut, Blutderivaten, Serum, Plasma, Urin, Liquor cerebrospinalis, Mundflüssigkeit, Synovialflüssigkeit und Emphysemflüssigkeit, wobei das Verfahren Folgendes beinhaltet: das Bereitstellen eines homogenen Analysenformats; das In-Kontakt-Bringen der Probe mit mindestens einem Reagens, das mindestens einen spezifischen Binder für Anti-CCP-Antikörper, immobilisiert an monodispersen Metall- oder Polymernanopartikeln mit einem Durchmesser von zwischen 10 und 250 nm, beinhaltet, um eine Lösung oder Suspension eines Anti-CCP-Bindungspartnerkomplexes in einer homogenen Probenmischung zu bilden; und das Detektieren der Gegenwart oder des Spiegels des Anti-CCP-Bindungspartnerkomplexes in einer homogenen flüssigen Phase, wobei die Detektion mittels Turbidimetrie durchgeführt wird, wobei in dem Verfahren die Probe und mindestens ein Reagens gemischt werden, ein Signal erzeugt wird und dieses Signal ohne Trennungs- oder Waschschritte, die eine Phasentrennung umfassen, detektiert wird und wobei der Binder für Anti-CCP mindestens ein CCP-Peptidantigen ist, wo zwei oder mehrere solcher Peptide an jeden Partikel gebunden sind.

2. Verfahren zur Bewertung des Vorliegens von, Risikos für, Potenzials für oder der Neigung zu rheumatoider Arthritis (RA) in einem Individuum, wobei das Verfahren das Analysieren auf Anti-CCP in einer körperlichen Probe von dem Individuum in einer homogenen Analyse gemäß Anspruch 1 beinhaltet, anhand welcher der Spiegel von Anti-CCP-Antikörpern in der Probe bestimmt wird, und das Korrelieren des somit bestimmten Spiegels mit dem Vorliegen von, Risiko für, Potenzial für oder der Neigung zu RA in dem Individuum beinhaltet, wobei das Vorliegen von Anti-CCP oder einer Konzentration von Anti-CCP oberhalb eines oder mehrerer Schwellenwerte mit dem Vorliegen von, erhöhten Schweregrad von, erhöhten Risiko für, erhöhten Potenzial für und/oder der erhöhten Neigung zu RA korreliert, und das Nichtvorliegen von Anti-CCP oder eine Konzentration von Anti-CCP unterhalb eines oder mehrerer Schwellenwerte mit dem Nichtvorliegen von, verminderten Schweregrad von, verminderten Risiko für, verminderten Potenzial für und/oder der verminderten Neigung zu RA korreliert, wobei der Schwellenwert ein solcher ist, dass 80% der Patienten mit klinisch bestätigter RA Proben bereitstellen werden, die ein Signal oberhalb dieses Werts erzeugen, und wobei eine Konzentration des Anti-CCP oberhalb dieser Schwellenwerte mit dem Vorliegen von, erhöhten Risiko für, erhöhten Potenzial für und/oder der erhöhten Neigung zu RA korreliert.

3. Verfahren gemäß Anspruch 2, das die Bewertung anderer biochemischer Marker, wie etwa Rheumafaktor (RF), in Kombination mit der Bewertung von Anti-CCP einschließt.

4. Verfahren gemäß einem der Ansprüche 1 bis 3, wobei der spezifische Binder für Anti-CCP-Antikörper an mindestens einen signalerzeugenden Anteil gebunden ist, wobei vorzugsweise ein detektierbares Signal durch die Bildung eines Komplexes erzeugt wird, der den signalerzeugenden Anteil und mindestens einen anderen signalerzeugenden Anteil des gleichen oder eines verschiedenen Typs enthält.

5. Verfahren gemäß Anspruch 4, wobei der signalerzeugende Anteil ein Nanopartikel ist, wobei vorzugsweise der Nanopartikel an mehr als einen spezifischen Binder für Anti-CCP-Antikörper gebunden ist.

6. Verfahren gemäß einem der Ansprüche 1 bis 5, wobei das Verfahren zusätzlich den Schritt des Zugebens eines Trübungsverstärkers beinhaltet.

7. Verfahren gemäß einem der Ansprüche 1 bis 6, das ferner das Durchführen des Verfahrens an Kalibrationsproben beinhaltet, die einen Anti-CCP-Gehalt von 0 bis 20 µg/ml an Anti-CCP-Antikörper aufweisen.

8. Verfahren gemäß einem der Ansprüche 2 bis 7, wobei das Individuum ein Individuum ist, das aus der Gruppe von Individuen ausgewählt ist, die aus älteren Individuen, Individuen, die Knochenverletzungen oder-schäden erlitten haben, Individuen, deren Gelenke einen erhöhten oder abnormen Gelenkverschleiß erlitten haben, Individuen mit entzündlichen und/oder Autoimmunkrankheiten, Individuen mit einer familiären Krankengeschichte von RA, Individuen, bei denen ein Gentest durchgeführt wurde, der eine erhöhte Neigung zu RA angezeigt hat, und Individuen mit klinischen Symptomen, die eindeutig RA anzeigen, besteht.

9. Kit zur Durchführung der Verfahren gemäß einem der Ansprüche 1 bis 8, der mindestens einen spezifischen Binder für Anti-CCP-Antikörper, immobilisiert an monodispersen Metall- oder Polymernanopartikeln mit einem Durchmesser von zwischen 10 und 250 nm, beinhaltet, wobei der Binder für Anti-CCP mindestens ein CCP-Peptidantigen ist und wobei zwei oder mehrere solche Peptide an jeden Partikel gebunden sind.

10. Kit gemäß Anspruch 9, wobei die Bindung des homogenen spezifischen Binders und der Anti-CCP-Antikörper reagiert, um ein Signal zu erzeugen.

11. Kit gemäß Anspruch 10, der ferner mindestens einen der folgenden Bestandteile beinhaltet:
mindestens einen signalerzeugenden Anteil;
mindestens Anti-CCP-Lösung mit bekannter Konzentration;
mindestens einen Trübungsverstärker;
ein lichtdurchlässiges Gefäß;
einen Detektor.

## Revendications

1. Procédé pour analyser des anticorps de peptides citrullinés anticycliques (anti-CCP) dans un échantillon clinique choisi entre le sang, des dérivés du sang, le sérum, le plasma, l'urine, le fluide cérébrospinal, un fluide oral, un fluide synovial et un fluide d'emphysème, ledit procédé comprenant la fourniture d'un format d'analyse homogène, la mise en contact dudit échantillon avec au moins un réactif comprenant au moins un liant spécifique pour anticorps anti-CCP immobilisés sur des nanoparticules métalliques ou polymères monodispersées ayant un diamètre compris entre 10 et 250 nm afin de former une solution ou une suspension d'un complexe partenaire de liaison anti-CCP dans un mélange d'échantillon homogène et détecter la présence ou le niveau dudit complexe partenaire de liaison anti-CCP dans une phase liquide homogène, dans lequel ladite détection est effectuée par turbidimétrie, dans lequel, dans ledit procédé, l'échantillon et au moins un réactif sont mélangés, un signal est généré et ce signal est détecté sans étapes de séparation ou de lavage quelconques impliquant une séparation de phases et dans lequel le liant pour l'anti-CCP est au moins un antigène de peptide CCP où deux de ces peptides ou plus sont liés à chaque particule.

2. Procédé pour l'évaluation de l'existence ; du risque ; du potentiel ; ou de la propension à une arthrite rhumatoïde (RA) chez un sujet, ledit procédé comprenant les étapes consistant à analyser l'anti-CCP dans un échantillon corporel venant dudit sujet au cours d'une analyse homogène selon la revendication 1, de manière à déterminer le niveau d'anticorps anti-CCP dans ledit échantillon, et à corréler le niveau ainsi déterminé avec l'existence ; le risque ; le potentiel ; ou la propension à une RA chez ledit sujet, dans lequel l'existence d'anti-CCP ou une concentration de peptides citrullinés anticycliques (anti-CCP) au-dessus d'une ou plusieurs valeurs de seuil est corrélée à l'existence, à la gravité accrue, au risque accru, au potentiel accru et/ou à la propension accrue à une RA, et la non-existence d'un anti-CCP ou une concentration d'anti-CCP en dessous d'une ou plusieurs valeurs de seuil est corrélée à la non-existence, à la gravité réduite, au risque réduit, au potentiel réduit et/ou à la propension réduite à une RA, dans lequel ladite valeur de seuil est telle que 80 % des patients ayant une RA cliniquement confirmée fournissent des échantillons générant un signal au-dessus de cette valeur et dans lequel une concentration d'anti-CCP au-dessus desdites valeurs de seuil est corrélée à l'existence, à un risque accru, à un potentiel accru et/ou à une propension accrue à une RA.

3. Procédé selon la revendication 2, comprenant l'évaluation d'autres marqueurs biochimiques, tels que le facteur rhumatoïde (RF), en combinaison avec l'évaluation d'un anti-CCP.

4. Procédé selon l'une quelconque des revendications 1 à 3, dans lequel ledit liant spécifique pour les anticorps anti-CCP est lié à au moins un radical générateur de signal, de préférence dans lequel un signal détectable est généré par la formation d'un complexe contenant ledit radical générateur de signal et au moins un autre radical générateur de signal du même type ou d'un type différent.

5. Procédé selon la revendication 4, dans lequel ledit radical générateur de signal est une nanoparticule, de préférence dans lequel ladite nanoparticule est liée à plus d'un liant spécifique pour l'anticorps anti-CCP.

6. Procédé selon l'une quelconque des revendications 1 à 5, ledit procédé comprenant en outre l'étape d'addition d'un promoteur d'opacité.

7. Procédé selon l'une quelconque des revendications 1 à 6, comprenant en outre la réalisation du procédé sur des échantillons d'étalonnage ayant des teneurs en anti-CCP de 0 à 20 µg/mL d'anticorps anti-CCP.

8. Procédé selon l'une quelconque des revendications 2 à 7, dans lequel ledit sujet est un sujet choisi dans le groupe de sujets constitué de sujets âgés, de sujets ayant souffert de lésions ou de dommages osseux, de sujets dont les articulations ont souffert d'usure accrue ou anormale, de sujets ayant des maladies inflammatoires et/ou auto-immunes, de sujets ayant un historique familial de RA, de sujets ayant eu un test génétique indiquant une plus grande propension à une RA et de sujets ayant des symptômes cliniques qui indiquent sans équivoque la RA.

9. Kit pour réaliser les procédés selon l'une quelconque des revendications 1 à 8, comprenant au moins un liant spécifique pour un anticorps anti-CCP immobilisé sur des nanoparticules métalliques ou polymères monodispersées ayant un diamètre compris entre 10 et 250 nm, dans lequel le liant pour l'anti-CCP est au moins un antigène de peptide CCP et dans lequel deux ou plusieurs de ces peptides sont liés à chaque particule.

10. Kit selon la revendication 9, dans lequel la liaison du liant spécifique homogène et de l'anticorps anti-CCP se fait pour générer un signal.

11. Kit selon la revendication 10, comprenant en outre au moins l'un des constituants suivants :
au moins un radical générateur de signal ;
au moins une solution anti-CCP de concentration connue ;
au moins un promoteur d'opacité ;
un récipient transmetteur de lumière ; et
un détecteur.
